Europäisches Patentamt

(19) European Patent Office

Office européen des brevets

(11) Publication number: **0 218 679 B1**

(12) # EUROPEAN PATENT SPECIFICATION

(45) Date of publication of patent specification:
**11.12.91 Bulletin 91/50**

(51) Int. Cl.⁵: **A61K 9/12, A61K 31/68**

(21) Application number: **86902640.1**

(22) Date of filing: **02.04.86**

(86) International application number:
**PCT/US86/00665**

(87) International publication number:
**WO 86/05987 23.10.86 Gazette 86/23**

(54) AEROSOL COMPOSITIONS FOR NASAL DELIVERY OF VITAMIN B 12.

The file contains technical information
submitted after the application was filed and
not included in this specification

(30) Priority: **16.04.85 US 723859**

(43) Date of publication of application:
**22.04.87 Bulletin 87/17**

(45) Publication of the grant of the patent:
**11.12.91 Bulletin 91/50**

(84) Designated Contracting States:
**AT BE CH DE FR GB IT LI LU NL SE**

(56) References cited:
**US-A- 2 746 796
US-A- 2 914 222
US-A- 4 525 341**

(56) References cited:
**Monto et al: Am.J.Med.sci., 223, 113 (1953) p.
65-71 file
Monto et al.: Arch. of. Int. Med. 93/219 (1954) p.
72-83 file**

(73) Proprietor: **NASTECH PHARMACEUTICAL
COMPANY, INC.
800 Veterans Memorial Highway
Hauppauge, NY 11788 (US)**

(72) Inventor: **WENIG, Jeffrey
9 Dickens Avenue
Dix Hills, NY 11746 (US)**

(74) Representative: **Weinhold, Peter, Dr. et al
Patentanwälte Dr. V. Schmied-Kowarzik
Dipl.-Ing. G. Dannenberg Dr. P. Weinhold Dr. D.
Gudel Dipl.-Ing. S. Schubert Dr. P. Barz
Siegfriedstrasse 8
W-8000 München 40 (DE)**

Note: Within nine months from the publication of the mention of the grant of the European patent, any
person may give notice to the European Patent Office of opposition to the European patent granted.
Notice of opposition shall be filed in a written reasoned statement. It shall not be deemed to have been
filed until the opposition fee has been paid (Art. 99(1) European patent convention).

## Description

This invention is concerned with aerosol compositions for nasal administration of a vitamin $B_{12}$ to a human suffering a vitamin $B_{12}$ deficiency.

Cyanocobalamin is a vitamin $B_{12}$, and is one of the $B_{12}$ class of vitamins which includes vitamin $B_{12a}$ (hydroxocobalamin), vitamin $B_{12b}$ (aquacobalamin), vitamin $B_{12c}$ (nitrilocobalamin), coenzyme $B_{12}$ (5'-deoxyadenosine cobalamine) and methyl $B_{12}$ (methyl cobalamine). Cyanocobalamin is the principal member of the class, and the most widely employed in medicine. This invention will be described as it relates to cyanocobalamin, but those skilled in the art will recognize that the invention is applicable to the class.

Vitamin $B_{12}$ is an essential compound for normal growth, hematopoiesis, production of all epithelial cells and maintenance of myelin throughout the nervous system. It was first isolated from liver concentrate by Rickes and his coworkers in 1948 and structurally elucidated by Hodgkin and her coworkers in the late 1950's. It is currently commercially available as a tablet and as an injectable.

Therapeutically, vitamin $B_{12}$ is employed in the treatment of a variety of $B_{12}$ deficiency afflictions, principally anemias such as pernicious and diphyllobothrium latum. Although the minimum daily requirement of vitamin $B_{12}$ is approximately -.1ug, the generally prescribed initial therapeutic dose is 100 to 1000µg given intramuscularly. Maintenance therapy with vitamin $B_{12}$ is usually 100µg intramuscularly, monthly and must be continued for life.

Since pernicious anemia is often a disease of later years when many sufferers have reduced muscle mass or are atrophic, repeated intramuscular injections of vitamin $B_{12}$ can be inconvenient, painful and often require doctor's visits. In some cases at least in the early stages, hospitalization is required. As a result, there is a need for a more convenient, less painful and less expensive method of administering vitamin $B_{12}$, particularly one that would not require hospitalization or repeated physician contacts.

Unfortunately, up to the present time no efficient method of administering $B_{12}$ which will achieve therapeutically useful blood levels of the vitamin except parenteral administration has been devised.

In 1953 and 1954 Monto et al in Am. J. Med. Sci., 223, 113 (1953) and Arch. of Int. Med. 93,219 (1954) described administration of $B_{12}$ by inhalation and nasal instillation. The vehicles for administration were aqueous isotonic sodium chloride solution and lactose powder. Although the results were reported as effective, safe and economical, the fact is that parenteral administration remains the only method regarded by the medical community as a safe, reliable and effective method for treating vitamin $B_{12}$ deficiencies in humans. No composition for nasal inhalation or instillation has become commercially available for nasal administration to mammals. There have been no published descriptions of compositions for nasal administration of a vitamin $B_{12}$ by aerosol techniques of which applicant is aware.

The difficulty with nasal instillation by nasal dosage as the procedure is described in the cited articles is that most of the $B_{12}$ passes immediately into the throat. It is not in contact with the nasal mucosa for a sufficient period of time to permit useful and uniform absorption. Most of the $B_{12}$ so administered is, in fact wasted.

Aerosol compositions have now been discovered for the nasal administration of $B_{12}$ in contact with the nasal mucosa for an extended period of time. During the time the compositions are in such contact, the $B_{12}$ is uniformly absorbed from the compositions through the nasal mucosa and is then uniformly distributed systemically. The use of the compositions, because of the efficiency with which the $B_{12}$ is absorbed allows the use of much lesser amounts of $B_{12}$ than is normally present in parenteral $B_{12}$ compositions. Moreover, since the patient can self administer the $B_{12}$, the need for hospitalization or physician contacts is minimized and may even be eliminated.

## THE INVENTION

This invention provides vitamin $B_{12}$ containing aerosol compositions specifically formulated for nasal administration which will retain the $B_{12}$ in contact with the nasal mucosa for a sufficiently long period of time to permit consistent, continuous and uniform absorption of therapeutically effective amounts of a vitamin $B_{12}$ through the nasal mucous membrane.

The invention, therefore comprises a therapeutic composition in aerosol form for nasal administration and absorption essentially through the nasal mucous membrane comprising a therapeutically effective amount of a vitamin $B_{12}$ in an isotonic aqueous buffer at a pH of from 4 to 6 in an aerosol formulation in a sealed container equipped with a metering valve which when actuated provides a spray of particles in which the particle size is from 5 to 50 microns, each separate spray containing from 50 to 1000 micrograms of a vitamin $B_{12}$.

The pH of the compositions of the invention is from 4 to 6. At this pH, $B_{12}$ is stable so that the compositions have a shelf life which may be a year or more. Additionally, at this pH, irritation of the nasal mucosa is minimal. The pH is maintained with a physiologically acceptable buffer composition suitably an acetate, phosphate, phthalate, borate, or other buffer.

2

An acetate buffer is preferred for convenience and economy.

The isotonicity of the composition is accomplished using sodium chloride, or other pharmaceutically acceptable agent such as dextrose, boric acid, sodium tartrate or other inorganic or organic solute. Sodium chloride is preferred particularly for buffers containing sodium ions.

The compositions of this invention may contain a humectant to inhibit drying of the mucous membrane and to prevent irritation. Any of a variety of humectants can be employed including, for example sorbitol, propylene glycol or glycerol. The concentration will vary with the selected agent, although the presence or absence of these agents, or their concentration is not an essential feature of the invention.

An enhanced absorption of $B_{12}$ across the mucous membrane may be accomplished employing a surfactant. Typically useful surfactants for these therapeutic compositions include polyoxyethylene derivatives of fatty acid partial esters of sorbitol anhydrides such as Tween®80, Polyoxyl 40 Stearate, Polyoxyethylene 50 Stearate and Octoxynol®. The usual concentration is from 1% to 10% based on the total weight.

A preservative may be employed to increase the shelf life of the compositions. Benzyl alcohol is suitable, although a variety of preservatives including, for example, Parabens, thimerosal, chlorobutanol, or benzalkonium chloride may also be employed. A suitable concentration of the preservative will be from 0.02% to 2% based on the total weight, although there may be appreciable variation depending upon the agent selected.

The compositions of the invention are dispensed from a sealed container equipped with a metering valve which when actuated releases a spray in which the particle size of the spray droplets is from 5 to 50 microns, preferably 10 to 20 microns. It has been found that if the spray droplets are below this range, they go directly through the nasal passages into the lungs. If they are larger, they coalesce into large drops which either run out of the nose or down into the throat.

Suitable containers and metering values are available commercially and need not be described here. They are available for use in packaging systems which deliver the aerosol compositions by all of the conventional aerosol techniques. These include mechanical pumps in which delivery is made by movement of a piston; compressed air mechanisms in which delivery is made by hand pumping air into the container; compressed gas techniques in which delivery is made by the controlled release of a compressed gas in the sealed composition; and liquid propellant techniques in which a low boiling liquid hydrocarbon or halohydrocarbon is vaporized to exert a pressure and force the aerosol composition through the metered valve. All of these systems are useful in the practice of this invention.

The most widely employed compressed gas for delivering aerosol compositions is nitrogen. The principal hydrocarbon is butane, although other low boiling hydrocarbons can be used in pure or mixed form. Fluorocarbons of the Freon® series are useful in the invention. These include, for example, Freon® 11, 12 and 14 and Fluorocarbon-FC152A.

All of the foregoing systems and propellants are useful for the nasal administration of the aerosol compositions of this invention.

Due to the efficiency with which $B_{12}$ is absorbed from the compositions of this invention, a therapeutically effective amount of $B_{12}$ for nasal administration will normally be appreciably less than for conventional methods of administration. Typically the concentrations of $B_{12}$ in the compositions of this invention will be from 0.05% to 1% by weight based on the total weight. The concentration may vary considerably however with the selected method of delivery. If the composition is a simple aqueous solution of $B_{12}$, possibly including excipients in solution or suspension under a compressed gas, the preferred concentration will be within the above range. But if the composition also contains propellants, the concentration of $B_{12}$ might vary. The important point is that the concentration be selected so that, acting together with the selected metering valve, each spray will deliver a dosage unit of from 50 to 1000 micrograms. It is of course possible to design an equivalent combination of concentration and metering valves so that a dosage unit containing 50 to 1000 micrograms of $B_{12}$ is delivered by two, three or even more valve actuations and resulting sprays.

The following aerosol compositions of this invention are useful for delivery by compressed gas systems or by mechanical pumps.

| Benzalkonium Chloride NF | 0.020 g |
| Thimerosal USP | 0.002 g |
| Acetic Acid NF | 0.100 g |
| Sodium Acetate (Anhydrous) USP | 0.270 g |
| Sodium Chloride USP | 0.820 g |
| Cyanocobalamin USP | 0.200 g |
| Water, Purified USP | q.s. 100.000 ml |

| Phenylmercuric Acetate NF | 0.002 g |
| Acetic Acid NF | 0.100 g |
| Sodium Acetate (Anhydrous) USP | 0.270 g |
| Boric Acid NF | 1.740 g |
| Cyanocobalamin USP | 0.500 g |
| Water, Purified USP | q.s. 100.000 ml |

| Benzalkonium Chloride NF | 0.020 g |
| Phenylmercuric Acetate NF | 0.002 g |
| Acetic Acid NF | 0.100 g |
| Sodium Acetate (Anhydrous) USP | 0.270 g |
| Boric Acid NF | 1.740 g |
| Cyanocobalamin USP | 1.000 g |
| Water, Purified USP | q.s. 100.000 ml |

Other compositions of this invention are produced by dissolving the $B_{12}$ in a solvent which is miscible with the selected propellant and taking the solution up in the propellant. The resulting solution is sealed in an appropriate container having a metered valve. Suitable solvents include, for example, ethylene glycol and polyethylene glycol. When the valve is acuated the $B_{12}$ is expelled in the solution and deposits on the nasal mucosa.

## Claims

1. A therapeutic composition in aerosol form for nasal administration and absorption essentially through the nasal mucous membrane comprising a therapeutically effective amount of a vitamin $B_{12}$ in an isotonic aqueous buffer at a pH of from 4 to 6 in an aerosol formulation in a sealed container equipped with a metering valve which when actuated provides a spray of particles in which the particle size is from 5 to 50 microns, each separate spray containing from 50 to 1000 micrograms of a vitamin $B_{12}$.

2. A therapeutic composition of claim 1 wherein the vitamin $B_{12}$ is cyanocobalamin.

3. A composition of claim 1 wherein the spray particle size is 10 to 20 microns.

## Patentansprüche

1. Therapeutische Zusammensetzung in Aerosolform zur nasalen Verabreichung und zur Absorption hauptsächlich durch die Nasenschleimhaut, welche eine therapeutisch wirksame Menge eines Vitamins $B_{12}$ in einem isotonischen wäßrigen Puffer mit einem pH-Wert von 4 bis 6 in einer Aerosolformulierung in einem verschlossenen Behälter umfaßt, der mit einem Meßventil versehen ist, das bei Betätigung einen Sprühstoß von Partikeln erzeugt, in dem die Partikel eine Größe von 5 bis 50 µm besitzen, wobei jeder einzelne Sprühstoß

4

EP 0 218 679 B1

50 bis 1000 µg eines Vitamins $B_{12}$ enthält.

2. Therapeutische Zusammensetzung gemäß Anspruch 1, in der das Vitamin $B_{12}$ Cyanocobalamin ist.

3. Zusammensetzung gemäß Anspruch 1, in der die Teilchengröße im Sprühstoß 10 bis 20 µm beträgt.

**Revendications**

1. Une composition thérapeutique sous forme d'aérosol en vue d'une administration et d'une absorption nasale essentiellement à travers la membrane muqueuse nasale , comprenant une quantité thérapeutiquement efficace d'une vitamine $B_{12}$ dans un tampon aqueux isotonique à un pH compris entre 4 et 6 dans une formulation d'aérosol dans un récipient fermé de façon étanche équipé d'une valve de dosage qui, lorsqu'elle est actionnée , fournit une pulvérisation de particules dans laquelle la dimension de particules est comprise entre 5 et 50 microns, chaque pulvérisation séparée contenant de 50 à 1000 microgrammes d'une vitamine $B_{12}$.

2. Une composition thérapeutique selon la revendication 1 dans laqsuelle la vitamine $B_{12}$ est la cyanocobalamine .

3. Une composition selon la revendication 1 dans laquelle la dimension de particules de pulvérisation est de 10 à 20 microns.